# EUROPEAN PATENT APPLICATION

(11) **EP 3 231 297 A1**
(43) Date of publication of application: **18.10.2017**
(21) Application number: 17460019.7
(22) Date of filing: 04.04.2017
(51) Int. Cl.: A23L 33/105, A23F 5/16, A61K 36/74

(54) **METHOD OF OBTAINING PREPARATIONS FROM UNROASTED COFFEE BEANS**

(30) Priority: 15.04.2016 PL 41685616
(71) Applicant: Mediam Chemical sp. z o.o., 91-078 Lodz (PL)
(72) Inventor: Cielecka, Izabela, 90-560 Lodz (PL); Kozniewski, Bartlemiej, 94-017 Lodz (PL); Gitner, Tomasz, 90-329 Lodz (PL)
(74) Representative: Dziubinska, Joanna

(57) **Abstract**

Method of obtaining preparations from unroasted coffee beans in which ground coffee is flooded with a mixture of alcohol and water, sulfuric acid is added, then heated to reflux, then cooled, filtered under reduced pressure, washed with a mixture of alcohol and water, the effluent is neutralized with sodium hydroxide and concentrated and then dried to give chlorogenic acid and caffeine, wherein the ground coffee is subjected to high temperature steam, then cooled and flooded with chloroform, then the chloroform is removed from the resultant mixture by filtration in vacuo and the ground grain is washed with chloroform and squeezed and then treated with steam at high temperature and flooded with a mixture of alcohol and water and sulfuric acid is added; then, in a known manner, the resulting mixture is heated to reflux, cooled, filtered under reduced pressure, and the grounds are washed with a mixture of alcohol and water and the liquid is neutralized with calcium hydroxide; the liquid is filtered, concentrated and dried to give chlorogenic acid and caffeine, and the resulting chloroform phase is concentrated and extracted and evaporated to give the green coffee oil and crystalline, white caffeine.

## Description

The subject of the documentation is the method of obtaining three formulations from unroasted coffee beans for use in food, pharmaceutical and cosmetic industries.

There are various known methods of processing coffee beans.

EP0107171 discloses a method for reducing the chlorogenic acid content in green coffee in which acidic substances are extracted from green coffee beans with an aqueous liquid and removed from the extract.

EP 1726213 describes a soluble coffee product that is made by treating the coffee extract with an enzyme.

WO 2006/108578 describes the method in which roasted coffee and green coffee beans are extracted to obtain a product of high antioxidant content.

FR 2734479 and EP 1674106 present the processes of extraction of green coffee beans in which organic solvents are used to obtain an extract enriched in bioactive ingredients without the typical coffee aroma.

Also known is the method in which the ground coffee is flooded with a mixture of ethanol and water, sulfuric acid is added, the mixture is heated to reflux, then the mixture is filtered under reduced pressure, the residue is washed with a mixture of ethanol and water, the effluent is neutralized with sodium hydroxide and concentrated. After drying, a precipitate is obtained which contains 90.7% chlorogenic acid and 6.2% caffeine and other unidentified substances.

The process according to the invention, by using a steaming-decaffeinating and oil removal-steaming-CQA extraction process leads to a higher CQA yield, higher purity, and two additional products, namely green coffee oil, which is a cosmetic raw material, and caffeine which is a food and pharmaceutical raw material. This process, due to the use of isopropanol, is cheaper than the prior art methods, and generates additional products.

The essence of the method of obtaining preparations from unroasted coffee beans according to the invention is that the ground coffee is subjected to high temperature steam, then cooled and flooded with chloroform, then the chloroform is removed from the resultant mixture by filtration in vacuo and the ground grain is washed with chloroform and squeezed and then treated with steam at high temperature and flooded with a mixture of alcohol and water and sulfuric acid is added; then, in a known manner, the resulting mixture is heated to reflux, cooled, filtered under reduced pressure, and the grounds are washed with a mixture of alcohol and water and the liquid is neutralized with calcium hydroxide; the liquid is filtered, concentrated and dried to give chlorogenic acid and caffeine, and the resulting chloroform phase is concentrated and extracted and evaporated to give the green coffee oil and crystalline, white caffeine.

The method of the invention bases on the fact that coffee should be ground to obtain grains of medium size, from 0.5 to 1.5 mm. The ground coffee is steamed at 90-120 ° C for a period of time of 20 to 60 minutes. The ground coffee is brought to room temperature and flooded with chloroform, dichloromethane or ethyl acetate, preferably chloroform, using 2-10 ml of solvent per gram of coffee. The suspension is left on a shaker, agitator or on table for 4-12 hours at 10-30 degrees. Then the solvent is removed by gravity filtration or under reduced pressure, the grains are washed several times with 0.1-1 ml of solvent per gram of coffee and squeezed. Solvent treated grains are treated with water vapor at a temperature of 90-120 °C for a period of 20-80 minutes, followed by flooding with a 1:1 to 98:2 ratio mixture of alcohol and water in an amount of 3 -10 ml per gram of coffee. The alcohol is selected from the group consisting of methanol, ethanol, n-propanol and isopropanol. Mixture is acidified with an inorganic acid, preferably sulfuric acid, to pH 1-4. The mixture is heated to a boil using appropriate laboratory equipment, at a normal pressure for 2-6 hours. The mixture is cooled, filtered with gravity or under reduced pressure, washed with several portions of the used alcohol/water mixture and neutralized with calcium hydroxide or magnesium hydroxide, preferably calcium hydroxide to a pH of about 6.5 - 7.5. The filtrate is filtered gravitationally or under reduced pressure, and concentrated in vacuo to 5-25% of the initial volume or until dry.

The solvent phase obtained in the first stage is concentrated to 10 - 50% volume and extracted 3-7 times with water portions equal to approximately the volume of solvent after evaporation, collecting the two phases. The solvent phase is evaporated in vacuo to give coffee oil, and after evaporation of the aqueous phase, crystalline caffeine is obtained.

The average yield with regards to raw coffee is 15-20% caffeoylquinic acids, 1-2% coffee oil, 2-4% caffeine.

The method of the invention is illustrated in non-limiting embodiments:

### Example 1:

150 g of green coffee was ground in a burr grinder to give ground coffee with a grain size of less than 1 mm. The ground coffee was steamed at 100 °C for 30 minutes. The ground coffee was cooled and 750 ml of chloroform was poured over it at room temperature. The mixture was stored on a laboratory table at room temperature for 8-10 hours. After this time, the chloroform was removed by filtration in vacuo, the grain was washed with 3 x 50 ml chloroform and squeezed. Chloroform-treated grains were subjected to steaming at 100 °C for 30 minutes and then flooded with a 1000 mL volume of mixture of 80% isopropanol and 20% water and sulfuric acid was added to a pH of about 3. The mixture was refluxed for 3 hours, then cooled, filtered under reduced pressure, the residue was washed with 3 x 50 ml of a 80% isopropanol 20% water mixture and neutralized with calcium hydroxide to pH 7. The effluent was filtered and concentrated to a volume of 100 ml (recovering about 750 mL of an azeotrope containing 90 % Isopropanol and 10% water) and then to dryness to give 28.7 g of dry, crystalline, yellowish residue. The residue contained 98.7% of chlorogenic acids (CQA) and 1.2% of caffeine and other unidentified substances. To increase stability, the solid was suspended in 121 mL of deionized water and neutralized with sodium hydroxide to give a clear solution of pH 7.2. The resulting chloroform phase was concentrated to 25% volume and extracted with 5 x 100 mL of water, collecting both phases. After evaporation of the chloroform phase, 1.9 g of green coffee oil was obtained, and after evaporation of the aqueous phase, 4.5 g of crystalline white caffeine was obtained. The oil sample was subjected to saponification with KOH in methanol / water, fatty acid extraction by acidification with HCl, and extraction of fatty acids into hexane. Fatty acids were analyzed by HPLC (palmitic acid: 26.8%, stearic acid 6.3%, oleic acid (omega 9): 8.2%, linoleic acid (omega 6): 52.2% linolenic acid 2.6%, residue - caffeine and polyphenols).

### Example2:

100 grams of green coffee was ground in a knife mill to give ground coffee with a grain size of about 0.8 mm. The ground coffee was steamed at 90 °C for 40 minutes. The ground coffee was cooled and flooded with ethyl acetate (500 mL) at room temperature. The mixture was stored on a stirrer at 30 ° C for 4 hours. After this time, ethyl acetate was removed by filtration in vacuo, the grain washed with 3 x 50 mL ethyl acetate and squeezed. Grain after treatment with ethyl acetate was subjected to steam at 80 °C for 40 minutes and then flooded with a mixture of 80% ethanol and 20% water in an amount of 750 ml. The mixture was refluxed for 4 hours, then cooled, filtered under reduced pressure, washed with 3 x 50 mL of 80% ethanol 20% water mixture, and the effluent was concentrated to 750 mL and then dryness to give 16,8 g of dry, crystalline, yellowish residue. This residue contained 95.7% chlorogenic acids (CQA) and 4% caffeine and other unidentified substances. To increase stability, the solid was suspended in 70 mL of deionized water and neutralized with sodium hydroxide to give a clear solution of pH 7.3. The resulting chloroform phase was concentrated to 25% volume and extracted with 5x500 mL of water, collecting both phases. After evaporation of the chloroform phase, 1.1 g of green coffee oil was obtained, and after evaporation of the aqueous phase, 2.5 g of crystalline white caffeine was obtained. An oil sample subjected to saponification with KOH in methanol / water, fatty acid extraction by acidification with HCl, and extraction of fatty acids into hexane.

## Claims

1. Method of obtaining preparations from unroasted coffee beans in which ground coffee is flooded with a mixture of alcohol and water, sulfuric acid is added, then heated to reflux, then cooled, filtered under reduced pressure, washed with a mixture of alcohol and water, the effluent is neutralized with sodium hydroxide and concentrated and then dried to give chlorogenic acid and caffeine, wherein the ground coffee is subjected to high temperature steam, then cooled and flooded with chloroform, then the chloroform is removed from the resultant mixture by filtration in vacuo and the ground grain is washed with chloroform and squeezed and then treated with steam at high temperature and flooded with a mixture of alcohol and water and sulfuric acid is added; then, in a known manner, the resulting mixture is heated to reflux, cooled, filtered under reduced pressure, and the grounds are washed with a mixture of alcohol and water and the liquid is neutralized with calcium hydroxide; the liquid is filtered, concentrated and dried to give chlorogenic acid and caffeine, and the resulting chloroform phase is concentrated and extracted and evaporated to give the green coffee oil and crystalline, white caffeine.
